# EUROPEAN PATENT SPECIFICATION

(11) **EP 1 174 521 B1**
(45) Date of publication and mention of the grant of the patent: **23.02.2005**
(21) Application number: 01116674.1
(22) Date of filing: 16.07.2001
(51) Int. Cl.: C12Q 1/68

(54) **Method for detecting transcribed genomic DNA sequences**
Methode zum Nachweis von transkribierten genomischen DNA-Sequenzen
Procédé de détéction de séquences d'ADN transcrites

(30) Priority: 14.07.2000 JP 2000218737; 28.08.2000 JP 2000263248; 30.10.2000 JP 2000334935
(43) Date of publication of application: 23.01.2002
(73) Proprietor: TOSOH CORPORATION, Shinnanyo-shi, Yamaguchi-ken (JP)
(72) Inventor: Ishiguro, Takahiko, Yokohama-shi, Kanagawa (JP); Yasukawa, Kiyoshi, Kawasaki-shi, Kanagawa (JP)
(74) Representative: VOSSIUS & PARTNER

(56) References cited:
- EP-A- 0 714 986
- EP-A- 0 969 101
- WO-A-99/10537
- HOCHGESCHWENDER U: "Toward a transcriptional map of the human genome." TRENDS IN GENETICS, vol. 8, no. 2, 1992, pages 41-44, XP001026355
- DATSON N A ET AL: "Scanning for genes in large genomic regions: cosmid based exon trapping of multiple exons in a single product" NUCLEIC ACIDS RESEARCH, OXFORD UNIVERSITY PRESS, SURREY, GB, vol. 24, no. 6, 1996, pages 1105-1111, XP002081565 ISSN: 0305-1048
- HOLZINGER I. ET AL.: "Cloning and genomic characterization of LST1: a new gene in the human TNF region." IMMUNOGENETICS, vol. 42, no. 5, 1995, pages 315-322, XP002187890
- ROMANO J W ET AL: "NASBA A NOVEL, ISOTHERMAL DETECTION TECHNOLOGY FOR QUALITATIVE AND QUANTITATIVE HIV-1 RNA MEASUREMENTS" CLINICS IN LABORATORY MEDICINE, W.B. SAUNDERS CO., LONDON, GB, vol. 16, no. 1, 1 March 1996 (1996-03-01), pages 89-103, XP000600141 ISSN: 0272-2712

### SEQUENCE LISTING

<110> Tosoh Corporation
   ISHIGURO, Takahiko
   YASUKAWA, Kiyoshi
<120> NOVEL GENOME ANALYZING METHOD
<130> PA210-0313
<150> JP 2000-218737
   <151> 2000-07-14
<150> JP 2000-263248
   <151> 2000-08-28
<150> JP 2000-334935
   <151> 2000-10-30
<160> 17
<170> Patentin version 3. 0
<210> 1
   <211> 53
   <212> DNA
   <213> Artificial
<220>
   <223>Primer 1F
<400> 1
   aattctaata cgactcacta tagggagatg cttccaagat tctggtggga ata 53
<210> 2
   <211> 20
   <212> DNA
   <213> Artificial
<220>
   <223> Primer 1R
<400> 2
   agtaagctaa taatgatgat 20
<210> 3
   <211> 35
   <212> DNA
   <213> Artificial
<220>
   <223> Primer 1S
<400> 3
   aagcatacaa tgtggagaca atgcataatc atcca 35
<210> 4
   <211> 53
   <212> DNA
   <213> Artificial
<220>
   <223> Primer 2F
<400> 4
   aattctaata cgactcacta tagggagagc ttttgatttt aacgactttt aac 53
<210> 5
   <211> 20
   <212> DNA
   <213> Artificial
<220>
   <223> Primer 2R
<400> 5
   tgtagtgttg actggagcag 20
<210> 6
   <211> 35
   <212> DNA
   <213> Artificial
<220>
   <223> Primer 2S
<400> 6
   aaagcttgtc aattggaacc agtcgcaatt atgaa 35
<210> 7
   <211> 53
   <212> DNA
   <213> Artificial
<220>
   <223> Primer 3F
<400> 7
   aattctaata cgactcacta tagggagaga agctgtcatc ggttactcag att 53
<210> 8
   <211> 20
   <212> DNA
   <213> Artificial
<220>
   <223> Primer 3R
<400> 8
   cctcttctcg agagataccc 20
<210> 9
   <211> 35
   <212> DNA
   <213> Artificial
<220>
   <223> Primer 3S
<400> 9
   gcttcagccg gaatttgtgc cgtttcatct tctgt 35
<210> 10
   <211> 53
   <212> DNA
   <213> Artificial
<220>
   <223> Primer 4F
<400> 10
   aattctaata cgactcacta tagggagatt ccggaagagc cccctcagca atg 53
<210> 11
   <211> 21
   <212> DNA
   <213> Artificial
<220>
   <223> Primer 4R
<400> 11
   ggactctctg ggaatactgg c 21
<210> 12
   <211> 35
   <212> DNA
   <213> Artificial
<220>
   <223> Primer 4S
<400> 12
   ccctccggga ctgctaactg gcaggagaac ttctg 35
<210> 13
   <211> 53
   <212> DNA
   <213> Artificial
<220>
   <223> Primer 5F
<400> 13
   aattctaata cgactcacta tagggagaga gggagacagc tctttctaca tag 53
<210> 14
   <211> 20
   <212> DNA
   <213> Artificial
<220>
   <223> Primer 5R
<400> 14
   ggggtttctg gccacggcag 20
<210> 15
   <211> 35
   <212> DNA
   <213> Artificial
<220>
   <223> Primer 5S
<400> 15
   ccctccggga ctgctaactg gcaggagaac ttctg 35
<210> 16
   <211> 20
   <212> DNA
   <213> Artificial
<220>
   <223> Oligonucleotide probe Y0-3
<400> 16
   cttctttagc agcaatgctg 20
<210> 17
   <211> 20
   <212> DNA
   <213> Artificial
<220>
   <223> Oligonucleotide probe Y0-3
<400> 17
   cagcattgct gctaaagaag 20

## Description

### FIELD OF THE INVENTION

This invention relates to a method for determining a gene expression region for a DNA sequence of which the nucleotide sequence is already known and a method for determining a gene expression region in an arbitrary region on a genome or the entire genome by repeatedly carrying out the above method. The invention also relates to a genomic gene which was determined to be a gene expression region by these methods and a protein encoded by the gene.

While nucleotide sequences of the genome in various biological species including the human genome composed of three billion bases are being revealed, development of so-called post-genome is in progress now. Accordingly, Hochgeschwender (1992), TIGS , 41-44, describes methods for identifying, in particular, transcribed genes within the human genome. Additionally, Datson (1996), Nucl. Acids Res. 24, 1105-1111, describes methods for screening gene fragments which may be used to detect the presence of a genomic sequence in a sample of RNA. Also Holzinger (1995), Immunogenetics 42, 315-322, describes methods for screening gene fragments. In particular, Holzinger (loc.cit.) describes the determination whether LST1, a DNA sequence known to be present in the human genome, is expressed. The target of post-genome is to understand kinds and activities of all proteins which are produced by a living thing during its entire life. Also, the main target for human post-genome is development of novel medicaments based on gene function analysis (creation of genomic drugs) and establishment of the basis of tailor-made medical treatments (cf., DeRisi *et al., Science,* vol. 278, p. 680, 1997).

In the post-genome, particularly the expression mode of RNA is called transcriptome. In transcriptome analysis, identification of all genes on the genome is an important subject. In this respect, EP-A1 0 969 101 discloses methods of assaying target nucleic acids which are useful for exploring unknown genes. Even if a genomic DNA sequence is revealed, each gene is not identified.

The number of genes on the human genome is estimated to be one hundred thousand, but only six thousand have so far been revealed. Even if some of the remaining genes have important roles, it is difficult to identify them.
(1) For example, in a two-dimensional electrophoresis, rare proteins are easily lost among housekeeping proteins existing in large amounts, so that their discrimination is practically impossible. Also, analysis of cDNA libraries has the same problem; namely, a probability of selecting rare cDNA and subjecting it to nucleotide sequence determination is extremely small. What is more, when identification of all genes is the target, the degree of accomplishment at present cannot be known by these methods.
(2) For example, micro-array is a technique to identify several thousand kinds of cDNA using tips to which they are linked, but since the linked cDNA molecules are already identified ones, conventionally unknown new genes are not identified.
(3) Also for example, an attempt to newly identify genes using a computer has been reported (cf., Bork *et al., Nature Genet.,* vol. 18, p. 313, 1998), and programs such as GRAIL, HEXON and GENSCAN are provided for carrying out this method. However, it goes without saying that identification of genes based on not assumptions but experimental data is strongly expected in the transcriptome analysis.

Thus, the object of the invention is to provide a novel transcriptome analysis method.

### SUMMARY OF THE INVENTION

A first embodiment of the invention made for achieving the above object is a method for determining whether or not a continued arbitrary DNA sequence existing in the genome of an arbitrary biological species, is a gene expression region, which comprises detecting whether or not a nucleotide sequence that corresponds to the nucleotide sequence of the region is present in the RNA of the biological species, wherein the detection is comprised of detecting whether or not DNA or RNA is amplified by the amplification of DNA or RNA based on the RNA of the biological species, using an oligonucleotide homologous to a sequence which is comprised of at least 10 or more continued bases and positioned in the 5'-end of the gene expression region and another oligonucleotide complementary to a sequence which is comprised of at least 10 or more continued bases and positioned in the 3'-end of the gene expression region and wherein the detection of whether or not DNA or RNA is amplified is carried out by a method in which the amplification is carried out in the presence of an oligonucleotide probe which can specifically bind to the DNA or RNA formed by the amplification and is labeled with an intercalating fluorescence dye (provided that the oligonucleotide is a sequence which does not form complementary bonding with any one of the aforementioned oligonucleotides), and changes in a fluorescence characteristic of the reaction solution is measured.

A second embodiment of the invention relates to the first invention, wherein the DNA sequence is a DNA region of from 100 to 200 bases.

A third embodiment of the invention relates to the preceding embodiment, wherein the amplification is an RNA amplification in which, using the oligonucleotides, either one of them having an RNA-transcriptable promoter sequence in its 5'-end, (1) a DNA fragment complementary to a part of RNA of the biological species is synthesized by RNA-dependent DNA polymerase from the either one of the oligonucleotides using the biological species-derived RNA as the template, thereby effecting formation of an RNA-DNA hybrid, (2) a single-stranded DNA fragment is formed by hydrolyzing the biological species-derived RNA of the RNA-DNA hybrid with ribonuclease H, (3) a DNA fragment complementary to the single-stranded DNA fragment is synthesized by DNA-dependent DNA polymerase from the other oligonucleotide using the single-stranded DNA fragment as the template, thereby effecting formation of a double-stranded DNA fragment having a promoter sequence capable of performing transcription of RNA as a part of the RNA of the biological species or RNA complementary to a part thereof, (4) an RNA transcription product is formed from the double-stranded DNA using RNA polymerase and then (5) the steps of from (1) to (4) are repeated using the RNA transcription product as the template.

A fourth embodiment of the invention relates to the preceding embodiments, wherein the probe can perform complementary bonding with at least a part of the sequence of the DNA transcription product or RNA transcription product formed by the amplification, and the fluorescence characteristic changes when compared with the case in which the complex is not formed.

A fifth embodiment of the invention is a method for determining the gene expression region in an arbitrary region on a genome or the entire genome, which comprises repeatedly carrying out the method of the first to fourth inventions.

### BRIEF DESCRIPTION OF THE INVENTION

Fig. 1 shows a relationship between the nucleotide sequence of each of the gene expression regions 1 to 5 and the complementary bonding position of each of the primers 1F, 1R, 1S, 2F, 2R, 2S, 3F, 3R, 3S, 4F, 4R, 4S, 5F, 5R and 5S.
Fig. 2 shows the non-transcription region and transcription region of the gene expression regions 1 to 5.
Fig. 3 shows an electrophoresis pattern when 30 cycles of RT-PCR was carried out for 200 ng of mRNA by the method shown in Example 3 using primers for the gene expression regions 1 to 5.
Figs. 4A, 4B, 4C show respective electrophoresis patterns when 10, 20 and 30 minutes of TRC was carried out for 200 ng of mRNA by the method shown in Example 4 using primers for the gene expression regions 1 to 5.
Fig. 5 is a graph showing a relationship between the reaction time and the fluorescence intensity ratio which increases with the formation of RNA, when TRC was carried out for 200 ng of mRNA by the method shown in Example 5 using primers for the gene expression region 3.
Figs. 6A and 6B show respective electrophoresis patterns when 30 cycles of RT-PCR or 30 minutes of TRC was carried out for 200 ng of mRNA by the method shown in Example 6 using primers for the gene expression region 3.
Figs. 7A and 7B show respective electrophoresis patterns when 30 cycles of RT-PCR or 30 minutes of TRC was carried out for 0 or 2 ng of mRNA and 0 to 200 ng of genomic DNA, by the method shown in Example 7 using primers for the gene expression region 3.
Fig. 8 is a graph showing a relationship between the reaction time and the fluorescence intensity ratio which increases with the formation of RNA, when TRC was carried out for 200 ng of genomic DNA by the method shown in Example 7 using primers for a region composed of the gene expression regions 1, 2 and 3.

### DETAILED DESCRIPTION OF THE INVENTION

The following describes the invention in detail.

The method of the invention is applied to a continued arbitrary DNA sequence existing in the genome of an arbitrary biological species. Such a DNA sequence, the length of which is not particularly limited but is 200 bases or less, preferably within the range of from 100 to 200 bases, can be set by selecting from published genomic DNA sequences. According to the method of the invention, a possibility that the DNA sequence is a gene expression region can be determined only in a case in which the entire portion of an arbitrarily set DNA sequence is included in one exon. Though the number of exons in a gene and the length of each exon greatly vary depending on the kind of gene, one exon containing a termination codon and a poly(A) connecting signal is present in every gene, which is longer than other exons and has more than 400 base pairs. Thus, when an arbitrary genomic region is fragmented into a DNA sequence of 200 base pairs or less, at least one of the fragments is included in the exon and therefore is not overlooked.

According to the present invention, the following detection is carried out by using a continued arbitrary DNA sequence existing in the genome, and the gene expression region can be determined on an arbitrary region in the genome or the entire genome by making the arbitrary region or the entire genome into fragments and repeating the detection using each fragment.

The invention determines whether or not a DNA sequence is a gene expression region by detecting the presence or absence of a nucleotide sequence which corresponds to the nucleotide sequence of the gene expression region, in RNA of the same biological species. The RNA to be used in the invention is mRNA which is prepared from the same biological species containing the genome to be determined for the gene expression region. Particularly, when the genome is a genome of a higher organism, it is desirable to use various types of mRNA, preferably prepared from all tissues. In that case, the mRNA may be used separately for each tissue or mixed. When the presence of a gene expression region is found in the latter case, subsequent separate use of mRNA of each tissue renders possible finding of a tissue which is expressing the gene in the genome determined to be the gene expression region. The reason that mRNA species can be mixed is as follows. Assuming that average molecular weight of mRNA is 300,000, 1 ng of mRNA will contain 2 x 10⁹ mRNA molecules. Accordingly, even in the case of a gene which is expressed in only one of 1,000 tissues and its expressing quantity is in a ratio of 1/100,000 of mRNA in the tissue, 2 x 10⁴ copies are present in 1 µg of the same amount mixture of mRNA respectively obtained from 1,000 tissues including this tissue. As will be described later in Examples, this copy number is sufficiently detectable.

Various methods can be applied to the above detection. For example, application of a hybridization method and a nucleic acid amplification method can be exemplified. When an amplification method is used, at least two oligonucleotides (primers) designed based on the gene expression region are used in both DNA amplification and RNA amplification, and one of them is an oligonucleotide homologous to a sequence which is comprised of at least 10 or more continued bases and positioned in the 5'-end of the gene expression region and the other is an oligonucleotide complementary to a sequence which is comprised of at least 10 or more continued bases and positioned in the 3'-end of the gene expression region. Oligonucleotides of at least 10 or more bases are used for keeping a specificity regarding binding of the oligonucleotides to the gene expression region.

Examples of the nucleic acid amplification method include a DNA amplification method typified by RT-PCR in which cDNA is synthesized from the mRNA using primers and a reverse transcriptase and then DNA (DNA comprised of the gene expression region) is amplified by a primer elongation reaction using the primers and a DNA polymerase and the DNA as the template, and an RNA amplification method in which cDNA complementary to the RNA is synthesized using primers and a reverse transcriptase and the mRNA as the template, an elongation reaction of DNA is carried out by binding it to a promoter primer having a moiety complementary to the DNA and then RNA (RNA comprised of the gene expression region) is synthesized in a large amount by allowing an RNA polymerase to react with the thus synthesized double-stranded DNA. The former case is an already broadly and generally known method, and examples of the latter case include NASBA (nucleic acid sequence based amplification) method, 3SR method and the method which will be described later in Examples.

In describing outlines of the NASBA method and the method described in Examples, they are RNA amplifications in which, using the oligonucleotides, either one of them having an RNA-transcriptable promoter sequence in its 5'-end, (1) a DNA fragment complementary to a part of RNA of the biological species is synthesized by RNA-dependent DNA polymerase from the either one of the oligonucleotides using the biological species-derived RNA as the template, thereby effecting formation of an RNA-DNA hybrid, (2) a single-stranded DNA fragment is formed by hydrolyzing the biological species-derived RNA of the RNA-DNA hybrid with ribonuclease H, (3) a DNA fragment complementary to the single-stranded DNA fragment is synthesized by DNA-dependent DNA polymerase from the other oligonucleotide using the single-stranded DNA fragment as the template, thereby effecting formation of a double-stranded DNA fragment having a promoter sequence capable of performing transcription of RNA as a part of the RNA of the biological species or RNA complementary to a part thereof, (4) an RNA transcription product is formed from the double-stranded DNA using RNA polymerase and then (5) the steps of from (1) to (4) are repeated using the RNA transcription product as the template.

The method described in Examples can be exemplified as particularly desirable detection method from the viewpoints that the determination of the invention can be effected within a short period of time because the amplification is completed within a markedly short time of 10 minutes, that it has a high sensitivity which enables amplification of even several pg of RNA containing the gene expression region and that the influence of DNA having a possibility of contaminating RNA can be excluded.

The DNA and RNA formed by the above amplification can be detected by an already known detection method such as an electrophoresis, but particularly preferred is a method in which the amplification is carried out in the presence of an oligonucleotide probe which can specifically bind to the DNA or RNA formed by the amplification and is labeled with an intercalating fluorescence dye, and changes in a fluorescence characteristic of the reaction solution is measured. As a matter of course, this probe is a sequence which does not form complementary bonding with the oligonucleotides used in the amplification. Examples of this oligonucleotide probe include those in which an intercalating fluorescence dye is linked to the phosphorus of an oligonucleotide via a linker. In the case of such a suitable probe, when the formed DNA or RNA forms double-strand by complementary bonding to a gene expression region (or a sequence complementary to the gene expression region), the intercalating fluorescence dye intercalates into the double-stranded moiety and changes its fluorescence characteristic, so that it is not necessary to separate the probe which did not form complementary bonding (Ishiguro, T. *et al.,* (1996), *Nucleic Acids Res.,* 24 (24), 4992 - 4997).

Nucleotide sequence of the oligonucleotide probe is not particularly limited with the proviso that it has a sequence that can perform complementary bonding with the formed DNA or RNA, but in order to keep a specificity regarding its bonding to the formed DNA or RNA, it is desirable that it has about 10 bases which are complementary to at least 10 continued bases existing in the DNA or RNA. In this connection, when the amplification is carried out in the presence of the oligonucleotide probe, it is desirable to modify the hydroxyl group of the 3'-end of the probe chemically (e.g., addition of glycolic acid) in order to suppress elongation reaction in which the probe is used as a primer.

When the amplification is carried out in the presence of the oligonucleotide probe as described above, the detection process of the invention can be carried out in one reaction container at a constant temperature and by one step, so that its application to automatic operation can be made easily.

Details of the genome analysis method of the invention which is carried out by repeating the gene expression region determination method are as follows, and the method can be applied to any biological species if the genomic sequence is determined. The genome of said biological species is divided, for example, into DNA sequences each having 200 base pairs. When a nucleic acid amplification is used as the detection method, a primer set containing two oligonucleotides necessary for amplifying each gene expression region is prepared. In this connection, the number of necessary primers and their sequences vary depending on the nucleic acid amplification method to be employed. Also, it is effective for improving working efficiency to exclude a DNA sequence which is present in a region already known as a gene expression region by previous studies and a DNA sequence which is present in a region that is obviously not a gene expression region based on its DNA sequence, from the objects. Next, the RNA is detected using a primer set for each DNA sequence.

When a genome is analyzed by the method of the invention, all genes of the biological species can be identified. In addition, it becomes possible to determine a protein encoded by a gene of interest, by isolating the gene determined to be a gene expression region and to produce the protein making use of the isolated DNA. For example, a nucleotide sequence can be determined by isolating complete length cDNA in the usual way using a nucleic acid amplified by the method of the invention as a probe. By doing this, the genomic structure including the relationship between intron and exon in the gene expression region is revealed. Also, a protein encoded by the gene can be known by isolating cDNA through the screening of a cDNA library in the usual way using the amplified nucleic acid as a probe. In addition, when this protein is expressed, it can be expressed by preparing a recombinant using the cDNA and using a microbial or animal cell as the host in the usual way.

Examples of the invention are given below by way of illustration and not by way of limitation.

### EXAMPLE 1

### Establishment of regions

In order to show realization possibility of the gene expression region determination method provided by the invention, the following model test was carried out.

As the genomic region, a region composed of 900 base pairs prepared from a G1 strain, a genetically engineered transformed methanol assimilating yeast strain which has been established by the method described by the present inventors in Japanese Patent Application No. 11-188650, was selected. When induced by methanol, the G1 strain expresses a human IL-6R-IL-6 fusion protein composed of one polypeptide chain of 397 amino acid residues (cf., Japanese Patent Publication No. P2001-8690A).

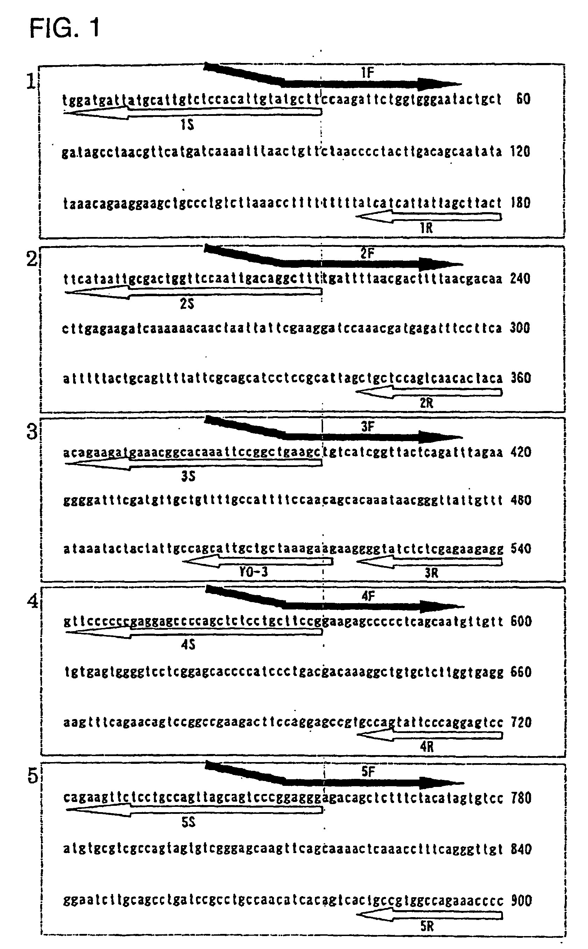

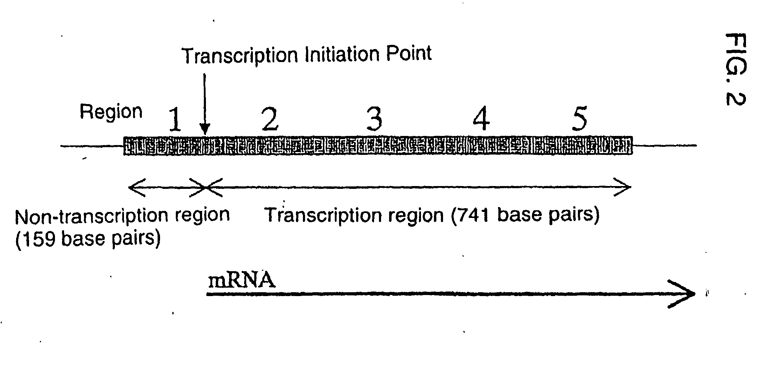

As shown in Fig. 1, the region composed of 900 base pairs was divided into five DNA sequences each having 180 base pairs. Also, mRNA expressing mode of the region already known from Japanese Patent Publication No. P2001-8690A is shown in Fig. 2. As is evident from Figs. 1 and 2, the gene expression region 1 (base numbers 1 to 180) contains 159 base pairs of a non-transcription region and 21 base pairs of a transcription region. Each of the gene expression region 2 (base numbers 181 to 360), gene expression region 3 (base numbers 361 to 540), gene expression region 4 (base numbers 541 to 720) and gene expression region 5 (base numbers 721 to 900) contains only a transcription region.

Oligonucleotide (primer) sets (forward primer; F, reverse primer; R, scissor probe; S) shown in Fig. 1 and SEQ ID NOs;1 to 15 were synthesized for each of the above five gene expression regions. When DNA amplification (RT-PCR) was carried out, the forward primer and reverse primer among them were used. When RNA amplification (TRC; transcription reverse transcription concerting amplification) was carried out, the forward primer, reverse primer and scissor probe were used. In the TRC, a gene expression region cannot be amplified when it is not located at the 5'-terminal of mRNA. The scissor probe is an oligonucleotide (DNA) to be used in that case for locating the gene expression region to the 5'-side of mRNA by complementarily binding it to the 5'-side of the gene expression region and cutting the complementarily bonded region by the action of a ribonuclease.

### EXAMPLE 2

### Preparation of mRNA

An mRNA sample of the strain G1 was prepared by the following method.

The strain was inoculated into 3 ml of BMGY (Bacto Yeast Extract 10 g/l, Bacto Peptone 20 g/l, Yeast Nitrogen Base without amino acids 1.34 g/l, 100 mM potassium phosphate buffer, pH 6.0, glycerol 10 g/l and biotin 0.4 mg/l) medium, and cultured at 28°C for 24 hours on a shaker.

A 100 µl portion of the culture broth was inoculated into 3 ml of BMGY (Bacto Yeast Extract 15 g/l, Bacto Peptone 30 g/l and other components having the same composition of the above BMGY) medium, and cultured at 28°C for 16 hours.

After confirmation of the depletion of methanol, 100 µl of methanol was added to the medium to induce expression of the human IL-6R-IL-6 fusion protein. Two hours after the addition of methanol, the cells were collected and 5 x 10⁷ of the cells were immediately frozen with liquid nitrogen.

They were subjected to cell wall lysis using a commercially available kit (Yeast cell lysis preparation kit, mfd. by BIO 01 Inc.) Next, mRNA was prepared using a commercially available kit (QuickPrep mRNA Purification Kit, mfd. by Amersham Pharmacia).

### EXAMPLE 3

### Determination of gene expression region by DNA amplification

Using the mRNA obtained in Example 2, examination was carried out on whether or not the DNA amplification is specific for a primer derived from a region composed solely of a gene expression region.

A commercially available kit (RT-PCR beads, mfd. by Amersham Pharmacia) was used in the RT-PCR.

That is, cDNA was synthesized from 200 ng of mRNA by a 15 minutes of reaction at 42°C using oligo(dT) as a primer. Next, PCR reaction was carried out using the forward primer and reverse primer. Using a thermal cycler, a cycle composed of 95°C for 1 minute, 55°C for 1 minute and 72°C for 2 minutes was repeated 30 cycles spending about 3 hours. Immediately after the reaction, an electrophoresis was carried out using 4% agarose which was then stained with SYBR Green.

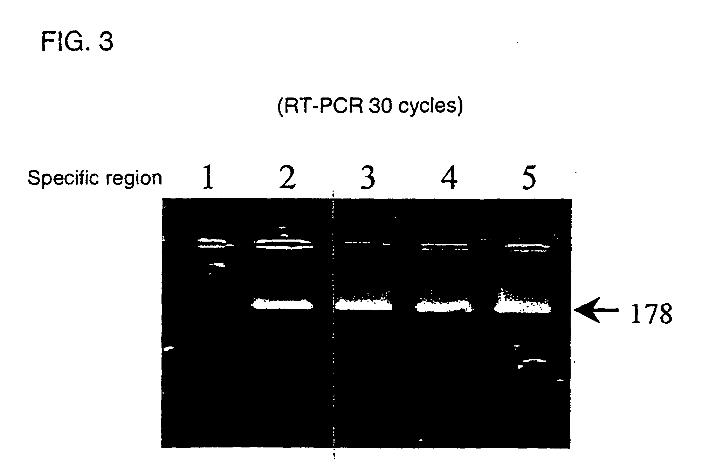

As is evident from Fig. 3, amplification was not found by the primer originated from the gene expression region 1 but was found by the primers originated from the gene expression regions 2 to 5.

These results show that the DNA amplification is specific for a primer derived from a region composed solely of a gene expression region, that is, whether or not a continued arbitrary DNA sequence existing in the genome of an arbitrary biological species, is a gene expression region can be determined by detecting the presence or absence of a nucleotide sequence which corresponds to the nucleotide sequence of the region in the RNA of the biological species, by a DNA amplification typified by RT-PCR.

### EXAMPLE 4

### Determination of gene expression region by RNA amplification

Using the mRNA obtained in Example 2, examination was carried out on whether or not the RNA amplification is specific for a primer derived from a region composed solely of a gene expression region.
(1) Using an RNA dilution solution (10 mM Tris-HCl (pH 8.0) and 1 mM EDTA), the sample was diluted to 200 ng/5 µl.
(2) A 20.8 µl portion of a reaction solution of the following composition was dispensed into 0.5 ml capacity tubes and 5 µl of the above RNA sample was added thereto.
   Reaction solution composition (each concentration is a concentration in 30 µl of the final reaction solution)
   60 mM of Tris-HCl (pH 8.6),
   13 mM of MgCl₂,
   90 mM of KCl,
   39 U of RNase inhibitor,
   1 mM of DTT,
   0.25 mM of each of dATP, dCTP, dGTP and dTTP,
   3.6 mM of ITP,
   3.0 mM of each of ATP, CTP, GTP and TTP,
   0.16 µM of scissor probe,
   1 µM of forward primer,
   1 µM of reverse primer,
   13% of DMSO, and
   distilled water for volume adjustment.
(3) This reaction solution was incubated at 65°C for 15 minutes and then at 41°C for 5 minutes, and then 4.2 µl of an enzyme solution having the following composition was added thereto.
   Enzyme solution composition (each concentration is a concentration in 30 µl of the final reaction solution)
   1.7% of sorbitol,
   3 µg of bovine serum albumin,
   142 U of T7 RNA polymerase (mfd. by Gibco),
   8 U of AMV reverse transcriptase (mfd. by Takara Shuzo),
   distilled water for volume adjusting use.
(4) Subsequently, the tubes were kept at 41°C for 10, 20 or 30 minutes. Immediately after the reaction, an electrophoresis was carried out using 4% agarose which was then stained with SYBR Green.

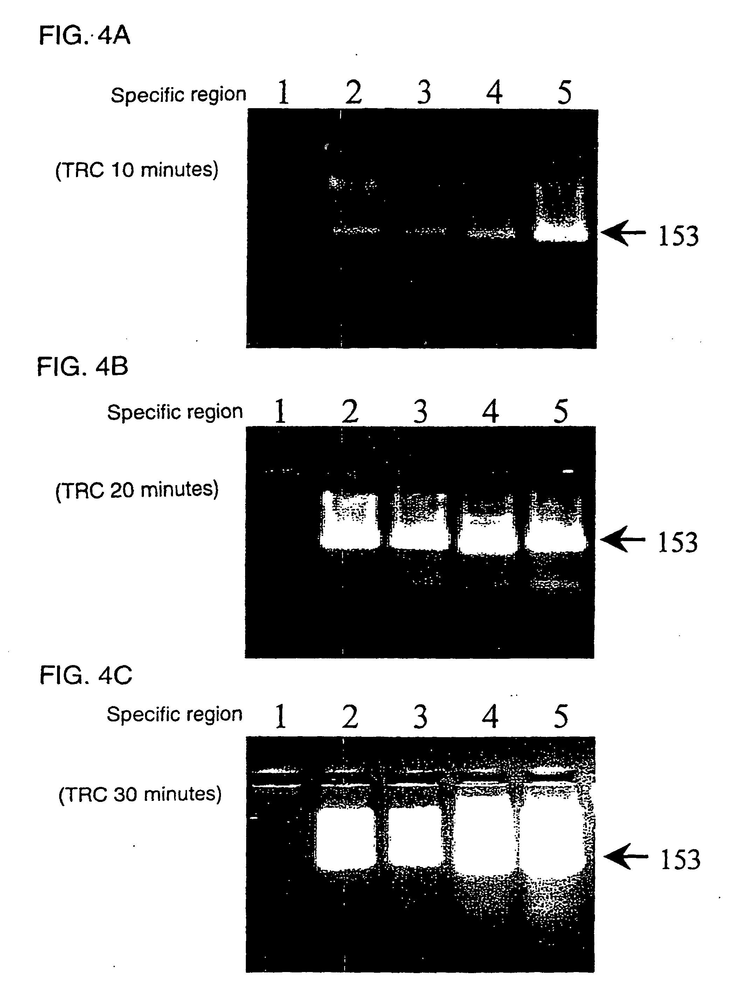

As is evident from Figs. 4A, 4B and 4C, amplification was not found when the primer for the gene expression region 1 was used in each case of the 10 minute reaction (Fig. 4A), 20 minute reaction (Fig. 4B) and 30 minute reaction (Fig. 4C), but was found when the primers for the gene expression regions 2 to 5 were used.

These results show that the RNA amplification is specific for a primer derived from a region composed solely of a gene expression region, that is, whether or not a continued arbitrary DNA sequence existing in the genome of an arbitrary biological species, is a gene expression region can be determined by detecting the presence or absence of a nucleotide sequence which corresponds to the nucleotide sequence of the region in the RNA of the biological species, by an RNA amplification typified by TRC.

Also, while the RT-PCR amplification shown in Example 3 required 3 hours even by the use of a thermal cycler, 10 minutes were enough for the amplification by TRC.

### EXAMPLE 5

### Measurement using oligonucleotide probe labeled with intercalating fluorescence dye

Using the mRNA obtained in Example 2, measurement using an oligonucleotide probe labeled with an intercalating fluorescence dye was carried out.
(1) Using an RNA dilution solution (10 mM Tris-HCl (pH 8.0) and 1 mM EDTA), the sample was diluted to 200 ng/5 µl.
(2) A 20. 8 µl portion of a reaction solution of the following composition was dispensed into 0.5 ml capacity tubes and 5 µl of the above RNA sample was added thereto.
   Reaction solution composition (each concentration is a concentration in 30 µl of the final reaction solution)
   60 mM of Tris-HCl (pH 8.6),
   13 mM of MgCl₂,
   90 mM of KCl,
   39 U of RNase inhibitor,
   1 mM of DTT,
   0.25 mM of each of dATP, dCTP, dGTP and dTTP,
   3.6 mM of ITP,
   3.0 mM of each of ATP, CTP, GTP and TTP,
   0.16 µM of scissor probe (3S, SEQ ID NO:9, the 3'-terminal hydroxyl group is aminated),
   1 µM of forward primer (3F, SEQ ID NO:7),
   1 µM of reverse primer (3R, SEQ ID NO:8),
   25 nM of an oligonucleotide labeled with an intercalating fluorescence dye (YO-3, SEQ ID NO;16, the intercalating fluorescence dye is labeled on the phosphorus between 6th position "T" and 7th position "T" counting from the 5'-terminal, and the 3'-terminal hydroxyl group is modified with glycol group),
   13% of DMSO, and
   distilled water for volume adjustment.
(3) This reaction solution was incubated at 65°C for 15 minutes and then at 41°C for 5 minutes, and then 4.2 µl of an enzyme solution having the following composition was added thereto.
   Enzyme solution composition (each concentration is a concentration in 30 µl of the final reaction solution)
   1.7% of sorbitol,
   3 µg of bovine serum albumin,
   142 U of T7 RNA polymerase (mfd. by Gibco),
   8 U of AMV reverse transcriptase (mfd. by Takara Shuzo)
   distilled water for volume adjusting use.
(4) Subsequently, the tubes were kept at 41°C and the reaction solution was periodically measured at an excitation wavelength of 470 nm and a fluorescence wavelength of 510 nm using a directly measuring fluorescence spectrophotometer equipped with a temperature controlling function.

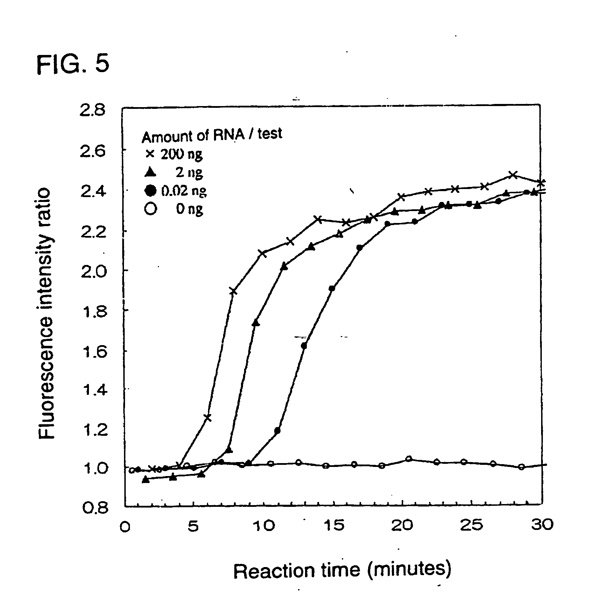

Periodical changes in the fluorescence intensity ratio of the sample (fluorescence intensity value at a predetermined time / background fluorescence intensity value) calculated by defining the time of the enzyme addition as 0 minute are shown in Fig. 5.

As shown in Fig. 5, the target RNA contained in 200 ng of mRNA was detected within about 6 minutes. In addition, the target RNA was detected within about 11 minutes even when the amount of mRNA was reduced to 0.02 ng. Thus, it was shown that quick and highly sensitivity measurement can be made by the use of an oligonucleotide probe labeled with an intercalating fluorescence dye.

### EXAMPLE 6

### Sensitivity

Sensitivities of RT-PCR and TRC were compared.

Using from 0 to 200 ng of the mRNA obtained in Example 2, amplification of DNA or RNA was carried out by 30 cycles of RT-PCR by the method shown in Example 3 or by 30 minutes of TRC by the method shown in Example 4.

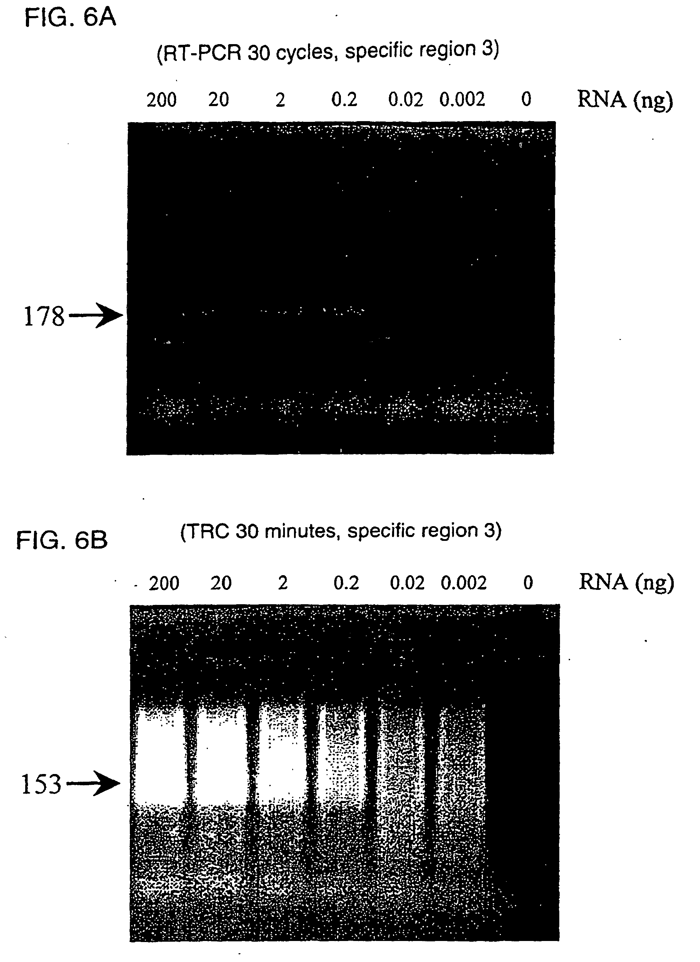

As is evident from Figs. 6A and 6B, amplification of 0.002 ng of the mRNA was not detected by RT-PCR but was detected by TRC. Thus, it was shown that TRC can achieve 10 times higher sensitivity than RT-PCR.

### EXAMPLE 7

### Influence of DNA contamination

Influences of the contamination of mRNA with DNA in RT-PCR and TRC were examined.

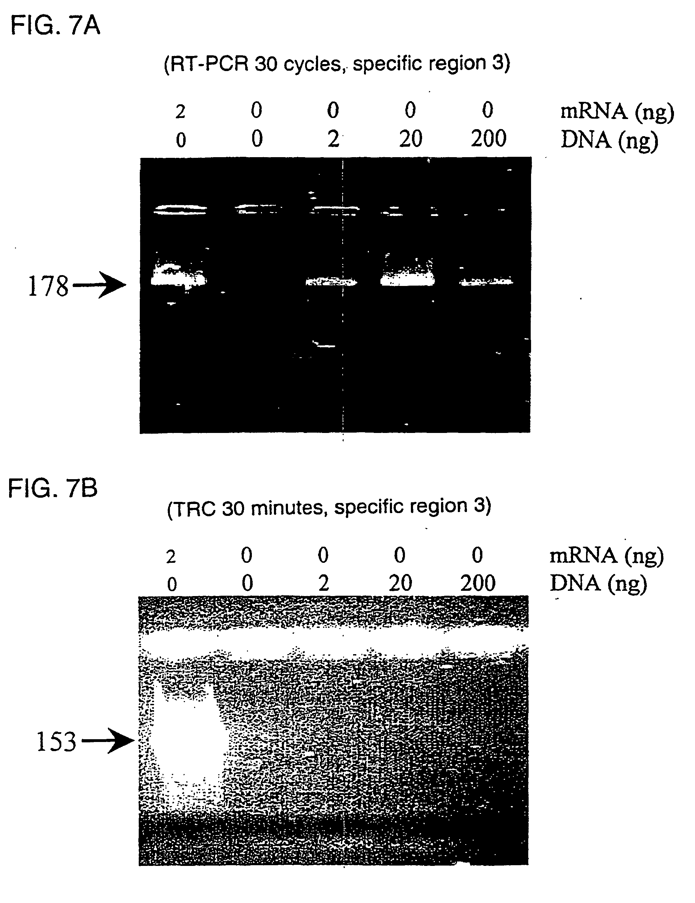

Firstly, using a commercially available kit (G Nome, mfd. by BIO 101 Inc.), genomic DNA was prepared from then cell wall-lysed G1 cell strain obtained by the method described in Example 1. Using from 0 to 200 ng of the DNA and 0 or 200 ng of the mRNA obtained in Example 2, 30 cycles of RT-PCR was carried out by the method shown in Example 3, and 30 minutes of TRC by the method shown in Example 4. As is evident from Figs. 7A and 7B, amplification was observed by RT-PCR when from 2 to 200 ng of the genomic DNA was present even in the absence of the mRNA. On the other hand, the amplification did not occur by TRC in the absence of the mRNA even when from 2 to 200 ng of the genomic DNA was present.

Next, relationship between denaturing condition and amplification of genomic DNA in TRC was examined. Using 200 ng of the genomic DNA, measurement by an oligonucleotide probe (YO-3, SEQ ID NO;16) labeled with an intercalating fluorescence dye was carried out by the method shown in Example 5. In this case, 1S (SEQ ID NO;3) was used instead of 3S (SEQ ID NO:9) as the scissor probe, and 1F (SEQ ID NO;1) was used instead of 3F (SEQ ID NO;7) as the forward primer. The reason for changing the scissor probe and forward primer is to prevent generation of amplification from RNA by changing the amplifying region to a region of 540 base pairs composed of the gene expression regions 1, 2 and 3 containing the 159 base pair non-transcription region. Also, the constant treating condition of the reaction solution before addition of the enzyme solution (incubation at 65°C for 15 minutes and then at 41°C for 5 minutes) was changed to the following three conditions.
(1) Incubation at 95°C for 15 minutes and then at 41°C for 5 minutes
(2) Incubation at 65°C for 15 minutes and then at 41°C for 5 minutes
(3) Incubation at 41°C for 5 minutes

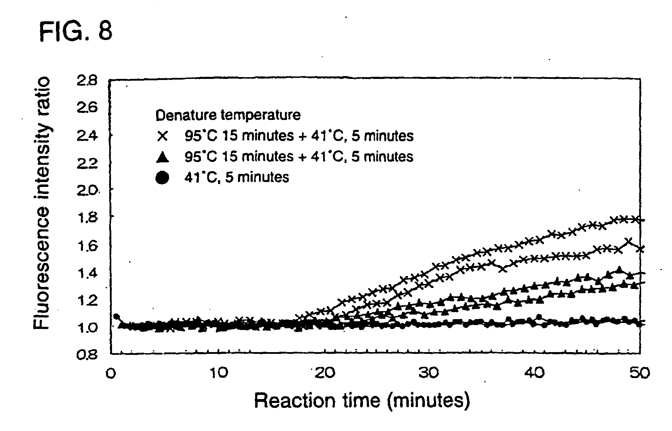

As is evident from Fig. 8, the time when the fluorescence intensity ratio exceeded 1.2 was about 28 minutes under the condition (1) and about 40 minutes under the condition (2), but its increase was not found under the condition (3).

This result shows that amplification can also occur from DNA by strengthening the denaturing condition. In this case, a change of the treating condition of the reaction solution before addition of the enzyme solution for the condition (3) is convenient in inhibiting amplification from DNA. However, it is expected that the amplification from RNA will be inhibited due to formation of the secondary structure of RNA. In addition, since periodical changes in the fluorescence intensity ratio are greatly different between the amplification from RNA and the amplification from DNA, it is markedly easy to make distinctions between both cases by comparing Fig. 5 with Fig. 8.

In summing up the above results, it is considered that a condition of incubating at 65°C for 15 minutes and then at 41°C for 5 minutes is appropriate as the treating condition of the reaction solution before addition of the enzyme solution in the mode for carrying out the invention.

Since the use of the method provided by the invention renders possible revelation of gene expression regions in the entire genome and also of the genomic stricture including the intron-exon relationship, sequences of all proteins capable of being expressed in an arbitrary biological species can be easily determined.

Consequently, according to the present invention, it is considered that understanding of all vital phenomena becomes possible by making rapid progress in the post-genome. Also, it is expected that the human post-genome will lead to the development of novel therapeutic and diagnostic drugs and also will greatly contribute to the progress of order-made medical treatments. It also will greatly contribute to the identification of industrially useful proteins from microorganisms living under extreme environmental conditions and to the application thereof.

While the invention has been described in detail and with reference to specific embodiments thereof, it will be apparent to one skilled in the art that various changes and modifications can be made therein.

## Claims

1. A method for determining whether or not a continued, arbitrary, known DNA sequence existing in the genome of an arbitrary biological species is a gene expression region, wherein said method comprises:
(a) detecting the presence or absence of a nucleotide sequence which corresponds to the DNA sequence of said gene expression region in the RNA of said biological species, wherein said detection comprises detecting whether or not DNA or RNA is amplified by carrying out amplification of DNA or RNA based on the RNA of said biological species, using an oligonucleotide homologous to a sequence which is comprised of at least 10 continued bases and positioned in the 5'-end of said gene expression region and another oligonucleotide complementary to a sequence which is comprised of at least 10 continued bases and positioned in the 3'-end of said gene expression region;
(b) carrying out the amplification in the presence of an oligonucleotide probe which can specifically bind to the DNA or RNA formed by the amplifcation and is labeled with an intercalating fluorescence dye, provided that said oligonucleotide is a sequence which does not form complementary bonding with any one of the aforementioned oligonucleotides; and
(c) measuring the change in a fluorescence characteristic of the reaction solution.

2. The method according to claim 1, wherein said gene expression region is a DNA region of from 100 to 200 bases.

3. The method according to claim 1 or 2, wherein at least one of said oligonucleotides has an RNA-transcriptable promoter sequence in its 5'-end and said amplification is an RNA amplification comprising:
(a) synthesizing a DNA fragment complementary to a part of RNA of said biological species by RNA-dependent DNA polymerase from said either one of the oligonucleotides using said biological species-derived RNA as the template, thereby effecting formation of an RNA-DNA hybrid;
(b) forming a single-stranded DNA fragment by hydrolyzing the biological species-derived RNA of said RNA-DNA hybrid with ribonuclease H;
(c) synthesizing a DNA fragment complementary to said single-stranded DNA fragment by DNA-dependent DNA polymerase from the other oligonucleotide using the single-stranded DNA fragment as the template, thereby effecting formation of a double-stranded DNA fragment having a promoter sequence capable of performing transcription of RNA as a part of the RNA of said biological species or RNA complementary to a part thereof;
(d) forming an RNA transcription product from said double-stranded DNA using RNA polymerase; and
(e) repeating the steps of from (a) to (d) using said RNA transcription product as the template.

4. The method according to any one of claims 1 to 3, wherein said probe is capable of performing complementary binding with at least a part of the sequence of the DNA transcription product or RNA transcription product formed by the amplification to change the fluorescence characteristic as compared with the case in which the complex is not formed.

5. A method for determining the gene expression region in an arbitrary region of a genome or the entire genome, which comprises repeatedly carrying out the method of any one of claims 1 to 4.

## Patentansprüche

1. Verfahren zum Feststellen ob eine durchgehende, beliebige, bekannte DNA-Sequenz, die im Genom einer beliebigen biologischen Art vorliegt, eine Genexpressions-Region ist oder nicht, wobei das Verfahren umfasst:
(a) Nachweisen der Anwesenheit oder Abwesenheit einer Nucleotidsequenz, die der DNA-Sequenz der Genexpressions-Region entspricht, in der RNA der biologischen Art, wobei der Nachweis das Feststellen umfasst, ob DNA oder RNA amplifiziert wird oder nicht, indem Amplifikation von DNA oder RNA, die auf der RNA der biologischen Art basiert, durchgeführt wird unter Verwendung eines Oligonucleotids, das homolog ist zu einer Sequenz, die mindestens zehn durchgehende Basen umfasst und im 5'-Ende der Genexpressions-Region liegt, und eines weiteren Oligonucleotids, das komplementär ist zu einer Sequenz, die mindestens zehn durchgehende Basen umfasst und im 3'-Ende der Genexpressions-Region liegt;
(b) Durchführen der Amplifikation in Gegenwart einer Oligonucleotidsonde, die spezifisch an die DNA oder RNA binden kann, die durch die Amplifikation gebildet wird und mit einem intercalierenden Fluoreszenzfarbstoff markiert ist, mit der Maßgabe, dass das Oligonucleotid eine Sequenz ist, die kein komplementäres Binden mit einem der vorstehend genannten Oligonucleotide zeigt; und
(c) Messen der Veränderung eines Fluoreszenzmerkmals der Reaktionslösung.

2. Verfahren nach Anspruch 1, wobei die Genexpressions-Region eine DNA-Region von 100 bis 200 Basen ist.

3. Verfahren nach Anspruch 1 oder 2, wobei mindestens eines der Oligonucleotide eine RNA-transkribierbare Promotorsequenz in seinem 5'-Ende hat und die Amplifikation eine RNA-Amplifikation ist, umfassend:
(a) Synthetisieren eines DNA-Fragments, das komplementär ist zu einem Teil der RNA der biologischen Art mittels RNA-abhängiger DNA-Polymerase von einem der beiden Oligonucleotide unter Verwendung der von der biologischen Art abgeleiteten RNA als Matrize, wodurch die Bildung eines RNA-DNA-Hybrides bewirkt wird;
(b) Bilden eines einzelsträngigen DNA-Fragments durch Hydrolysieren der von der biologischen Art abgeleiteten RNA des RNA-DNA-Hybrids mit Ribonuclease H;
(c) Synthetisieren eines DNA-Fragments, das komplementär zum einzelsträngigen DNA-Fragment ist, mittels DNA-abhängiger DNA-Polymerase vom anderen Oligonucleotid unter Verwendung des einzelsträngigen DNA-Fragments als Matrize, wodurch die Bildung eines doppelsträngigen DNA-Fragments bewirkt wird, das eine Promotorsequenz hat, die in der Lage ist, Transkription von RNA als Teil der RNA der biologischen Art oder von RNA, die komplementär zu einem Teil davon ist, durchzuführen;
(d) Bilden eines RNA-Transkriptionsprodukts von der doppelsträngigen DNA unter Verwendung von RNA-Polymerase; und
(e) Wiederholen der Schritte von (a) bis (d) unter Verwendung des RNA-Transkriptionsprodukts als Matrize.

4. Verfahren nach einem der Ansprüche 1 bis 3, wobei die Sonde in der Lage ist, komplementäres Binden mit zumindest einem Teil der Sequenz des DNA-Transkriptionsprodukts oder RNA-Transkriptionsprodukts durchzuführen, das durch die Amplifikation gebildet wurde, um das Fluoreszenzmerkmal im Vergleich mit dem Fall, in dem der Komplex nicht gebildet wird, zu verändern.

5. Verfahren zum Feststellen der Genexpressions-Region in einer beliebigen Region eines Genoms oder des gesamten Genoms, das das wiederholte Ausführen des Verfahrens nach einem der Ansprüche 1 bis 4 umfasst.

## Revendications

1. Procédé de détermination si oui ou non une séquence d'ADN connue, arbitraire, continue, existant dans le génome d'une espèce biologique arbitraire est une région d'expression d'un gène, dans lequel ledit procédé comprend les étapes consistant à :
(a) détecter la présence ou l'absence d'une séquence de nucléotides qui correspond à la séquence d'ADN de ladite région d'expression du gène dans l'ARN de ladite espèce biologique, dans lequel ladite détection comprend le fait de détecter si oui ou non l'ADN ou l'ARN est amplifié en conduisant l'amplification de l'ADN ou de l'ARN basée sur l'ARN de ladite espèce biologique, en utilisant un oligonucléotide homologue à une séquence qui se compose d'au moins 10 bases continues et positionnée à l'extrémité 5' de ladite région d'expression du gène et d'un autre oligonucléotide complémentaire d'une séquence qui se compose d'au moins 10 bases continues et positionnée à l'extrémité 3' de ladite région d'expression du gène ;
(b) conduire l'amplification en présence d'une sonde d'oligonucléotides qui peut spécifiquement se lier à l'ADN ou à l'ARN formé par l'amplification et qui est marquée à l'aide d'un colorant fluorescent intercalant, à condition que ledit oligonucléotide soit une séquence qui ne forme pas de liaison complémentaire avec l'un quelconque des oligonucléotides précédemment mentionnés ; et
(c) mesurer le changement dans une caractéristique de fluorescence de la solution réactionnelle.

2. Procédé selon la revendication 1, dans lequel ladite région d'expression du gène est une région d'ADN de 100 à 200 bases.

3. Procédé selon la revendication 1 ou 2, dans lequel au moins l'un desdits oligonucléotides a une séquence promotrice pouvant être transcrite par l'ARN dans sa terminaison 5' et ladite amplification est une amplification d'ARN comprenant les étapes consistant à :
(a) synthétiser un fragment d'ADN complémentaire d'une partie d'ARN de ladite espèce biologique par l'ADN polymérase ARN dépendante à partir de l'un ou l'autre desdits oligonucléotides en utilisant l'ARN dérivé de ladite espèce biologique comme matrice, effectuant par là la formation d'un hybride ARN-ADN ;
(b) former un fragment d'ADN simple brin par l'hydrolyse de l'ARN dérivé de l'espèce biologique dudit hybride ARN-ADN avec la ribonucléase H ;
(c) synthétiser un fragment d'ADN complémentaire dudit fragment d'ADN simple brin par l'ADN polymérase ADN dépendante à partir de l'autre oligonucléotide en utilisant le fragment d'ADN simple brin comme matrice, effectuant de là la formation d'un fragment d'ADN double brin ayant une séquence promotrice capable d'effectuer la transcription de l'ARN comme une partie de l'ARN de ladite espèce biologique ou de l'ARN complémentaire d'une partie de celle-ci ;
(d) former un produit de transcription de l'ARN à partir dudit ADN double brin en utilisant l'ARN polymérase ; et
(e) répéter les étapes de (a) à (d) en utilisant ledit produit de transcription de l'ARN comme matrice.

4. Procédé selon l'une quelconque des revendications 1 à 3, dans lequel ladite sonde est capable d'effectuer la liaison complémentaire avec au moins une partie de la séquence de du produit de transcription de l'ADN ou du produit de transcription de l'ARN formé par l'amplification pour modifier la caractéristique de fluorescence tel que comparé avec le cas dans lequel le complexe n'est pas formé.

5. Procédé pour déterminer la région d'expression du gène dans une région arbitraire d'un génome ou du génome entier, qui comprend le fait de conduire de manière répétée le procédé selon l'une quelconque des revendications 1 à 4.
